(19) Europäisches Patentamt
European Patent Office
Office européen des brevets

(11) Veröffentlichungsnummer : **0 159 586**
**B1**

(12) # EUROPÄISCHE PATENTSCHRIFT

(45) Veröffentlichungstag der Patentschrift : **30.11.88**

(51) Int. Cl.⁴ : **C 07 D249/08**, C 07 D233/62, A 01 N 43/653, A 01 N 43/50

(21) Anmeldenummer : **85103979.2**

(22) Anmeldetag : **02.04.85**

(54) **Azolverbindungen, Verfahren zu ihrer Herstellung und diese enthaltende Fungizide.**

(30) Priorität : **26.04.84 DE 3415486**

(43) Veröffentlichungstag der Anmeldung : **30.10.85 Patentblatt 85/44**

(45) Bekanntmachung des Hinweises auf die Patenterteilung : **30.11.88 Patentblatt 88/48**

(84) Benannte Vertragsstaaten : **BE CH DE FR GB IT LI NL**

(56) Entgegenhaltungen :
**EP-A- 0 040 350**
**EP-A- 0 069 290**
**EP-A- 0 100 952**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Schulz, Guenter, Dr.**
**Carl-Bosch-Strasse 96**
**D-6700 Ludwigshafen (DE)**
Erfinder : **Sauter, Hubert, Dr.**
**Neckarpromenade 20**
**D-6800 Mannheim 1 (DE)**
Erfinder : **Reissenweber, Gernot, Dr.**
**Drosselstrasse 15**
**D-6737 Boehl-Iggelheim (DE)**
Erfinder : **Ammermann, Eberhard, Dr.**
**Sachsenstrasse 3**
**D-6700 Ludwigshafen (DE)**



Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue, wertvolle Azolverbindungen, Verfahren zu ihrer Herstellung, diese Verbindungen enthaltende fungizide Mittel und ihre Verwendung als Fungizide.

Es ist bekannt, daß Imidazolderivate, zum Beispiel das 1-[2-(2,4-Dichlorphenyl)-2-(2-propenyloxy)-ethyl]-1H-imidazol (GB-PS 1 318 590) eine gute fungizide Wirksamkeit zeigen. Die Wirkung ist jedoch bei niedrigen Aufwandmengen und Anwendungskonzentrationen nicht immer befriedigend. Darüber hinaus ist die fungitoxische Wirkung oft mit einer hohen Phytotoxizität verbunden, so daß bei den für die Bekämpfung von Pilzen im Pflanzenschutz, beispielsweise bei der Bekämpfung von Rostpilzen notwendigen Konzentrationen auch die Kulturpflanzen geschädigt werden. Aus diesen Gründen sind sie für den Gebrauch als Pflanzenschutzmittel zur Bekämpfung von Pilzen zum Teil nicht geeignet.

Es ist ferner bekannt, Azolderivate, die einen Alkanrest enthalten, oder Azolylallylketone oder Azolylallylcarbinole als Fungizide zu verwenden (EP-A-40 350, EP-A-100 952).

Es wurde nun gefunden, daß Azolverbindungen der Formel

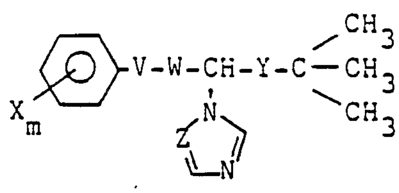
(I)

in welcher

V für Sauerstoff steht,

X für Wasserstoff, Fluor, Chlor, Brom, $C_1$-$C_4$-Alkyl, $C_1$-$C_4$-Alkoxy, Trifluormethyl, Phenyl oder Phenoxy steht,

m eine ganze Zahl von 1 bis 5 ist, wobei die einzelnen Atome oder Gruppen gleich oder verschieden sind, wenn m größer als 1 ist,

W für eine Gruppe der Formel $CH_2—CR^1 = CR^2—CH_2$ steht, wobei $R^1$ und $R^2$ gleich oder verschieden sind und Wasserstoff, Methyl, Ethyl, Chlor oder Fluor bedeuten,

Z für CH oder N steht,

Y für die Gruppe CHOH steht,

sowie deren für Pflanzen verträgliche Säureadditionssalze und Metallkomplexe eine sehr gute fungizide Wirkung bei ausgezeichneter Verträglichkeit bei Pflanzen haben.

In den Verbindungen der Formel I sind das azolylsubstituierte Kohlenstoffatom und das benachbarte durch Sauerstoff substituierte Kohlenstoffatom chiral. Die Wirkstoffe fallen demgemäß als Enantiomerengemische an, die in die optisch aktiven Enantiomere getrennt werden können. Im allgemeinen fallen die Wirkstoffe bedingt durch die Anwesenheit zweier chiraler Zentren auch als Diastereomerengemische an, die in üblicher Weise, z. B. durch Kristallisation oder Chromatographie in die einzelnen Komponenten getrennt werden können. Für die Anwendung der Verbindungen als Fungizide ist jedoch eine Trennung der Enantiomeren oder Diastereomeren normalerweise nicht erforderlich. Die Doppelbindung kann cis- oder trans-konfiguriert sein. Die Erfindung umfaßt sowohl die Gemische, auch die cis-trans-Gemische, als auch die sterisch oder optisch aktiven einheitlichen Substanzen.

Der Phenoxyrest kann beispielsweise fongendermaßen durch $X_m$ substituiert sein : Wasserstoff, 2-Fluor, 4-Fluor, 2-Chlor, 3-Chlor, 4-Chlor, 4-Brom, 2,4-Dichlor, 2,4,6-Trichlor, 2-Methyl, 3-Methyl, 4-Methyl, 4-n-Propyl, 4-tert.-Butyl, 4-n-Butyl, 3-Trifluormethyl, 4-Trifluormethyl, 4-Phenyl, 4-Methoxy, 4-Butoxy, 4-Phenoxy.

m bedeutet beispielsweise 1, 2 oder 3.

$R^1$ bedeutet beispielsweise Wasserstoff, Methyl oder Chlor.

$R^2$ bedeutet beispielsweise Wasserstoff, Methyl oder Chlor. Die Doppelbindung ist cis- oder trans-, vorzugsweise trans-konfiguriert.

Damit seien folgende Beispiele genannt :

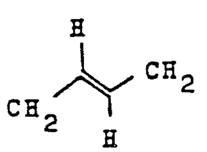 , 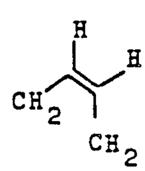 , 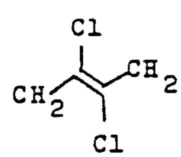 ,

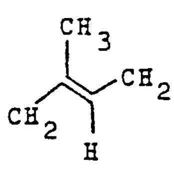 , 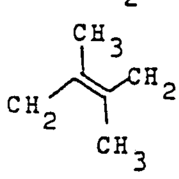 ,

Geeignete Säureadditionssalze sind beispielsweise die Chloride, Bromide, Sulfate, Nitrate, Phosphate, Acetate, Oxalate oder Dodecylbenzolsulfonate. Die Wirksamkeit der Salze geht auf das Kation zurück, so daß die wahl des Anions beliebig ist, sofern es nur für Pflanzen verträglich ist.

Geeignete Metallkomplexe sind Verbindungen der Formel

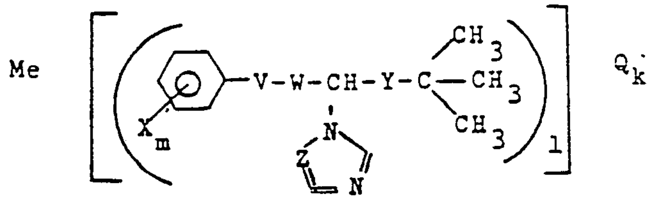 (V)

in der $X_m$, V, W, Z und Y die oben angegebene Bedeutung haben und Me ein Metall, z. B. Kupfer, Zink, Zinn, Mangan, Eisen, Cobalt oder Nickel bedeutet, Q für das Anion einer anorganischen Säure steht, z. B. Salzsäure, Schwefelsäure, Phosphorsäure oder Bromwasserstoffsäure und 1 und K 1, 2, 3 oder 4 bedeuten.

Die Azolylverbindungen der Formel I gemäß Anspruch 1, werden vorzugsweise durch stereoselektive Reduktion der Ketone der Formel

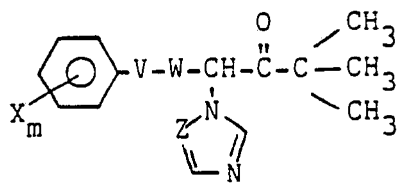 (II)

und gegebenenfalls anschließende Alkylierung oder Acylierung dargestellt.

Dabei entstehen bei der Reduktion mit komplexen Hydriden, vorzugsweise mit Natriumborhydrid in einem protischen Verdünnungsmittel, vorzugsweise Methanol, Ethanol oder i-Propanol, stark überwiegend die R*, R*-diastereomeren Alkohole der Formel I. Man erhält vorwiegend die diastereomeren Triazolylalkohole der R*, S*-Konfiguration, wenn man die Ketone der Formel II reduziert mit

a) mit sekundären Alkoholaten oder
b) mit Alkylmagnesiumhalogeniden oder
c) mit Wasserstoff in Gegenwart geeigneter Hydrierkatalysatoren.

Zur Erläuterung sei vermerkt, daß Verbindungen mit zwei verschiedenen chiralen Kohlenstoffatomen vier Stereoisomere bilden, deren Konfiguration nach dem Cahn-Ingold-Prelog-System bezeichnet werden kann (siehe z. B. D. Seebach und V. Prelog, Angew. Chem. 94, 696 (1982) und dort angegebene Literatur). Hierbei stellen zwei Enantiomere der Konfigurationen R, R und, S, S eine Diastereomer mit der Bezeichnung R*, R* dar. Das andere Diastereomer mit der Bezeichnung R*, S* setzt sich aus den beiden Enantiomeren mit den Konfigurationen R, S und S, R zusammen.

Die mit dem Reduktionsmittel a), b) oder c) erhältlichen Alkohole der Formel I mit Y = CHOH enthalten einen erheblich höheren Anteil der Diastereomeren mit R*,S*-Konfiguration als derjenigen mit R*,R*-Konfiguration. Der Anteil der R*,R*-Diastereomeren im Gemisch liegt in der Regel weit unter 30 %. Reine R*,S*-Diastereomere können daraus durch einfaches Waschen der Rohprodukte mit geeigneten Lösungsmitteln, wie z. B. Diisopropylether, oder durch Umkristallisieren oder durch andere, übliche Reinigungsschritte, z. B. Chromatographie erhalten werden.

Zur stereoselektiven Reduktion der Ketone der Formel II zu den R*,S*-Diastereomeren der Formel I mit Y = CHOH geht man nach a) folgendermaßen vor:

Die Ketone der Formel II (1 Moläquivalent) werden mit sekundären Alkoholaten (vorzugsweise 0,3 bis 1,5 Moläquivalente), vorzugsweise Alkoholaten des Aluminiums, z. B. Aluminiumisopropylat, Aluminiumbutylat-(2) oder Aluminiumcyclohexylat in Gegenwart eines Verdünnungsmittels bei 60 bis 160 °C, vorzugsweise bei der Siedetemperatur des Verdünnungsmittels umgesetzt. Als Verdünnungsmittel eignen sich inerte organische Lösungsmittel, insbesondere Alkohole wie Isopropanol oder Cyclohexanol. Die entstandenen diastereomeren Alkoholate werden sodann mit Hilfe von Säuren in üblicher Weise zu den freien Alkoholen der Formel I mit Y = CHOH hydrolysiert.

Für die stereoselektive Reduktion in der Ausführungsform b) des oben erwähnten Verfahrens werden die Ketone der Formel II (1 Moläquivalent) mit Alkylmagnesiumhalogeniden, die mindestens ein β-ständiges H-Atom enthalten, vorzugsweise mit 0,7-1,5 Moläquivalent, in Gegenwart eines Verdünnungsmittels bei Temperaturen zwischen 0 und 120 °C, umgesetzt. Als Alkylmagnesiumhalogenide kommen z. B. Ethylmagnesiumchlorid, Isopropylmagnesiumbromid, n-Propylmagnesiumbromid oder Isobutylmagnesiumchlorid in Frage. Als Lösungsmittel für die Reduktion kommen bevorzugt Ether in Frage, wie Diethylether, Di-n-propylether, Tetrahydrofuran oder Anisol, ferner tertiäre Amine wie N,N-Diethylanilin sowie Phosphorsäure-tris-(dimethylamid). Vorzugsweise kann man die Reaktion auch im Gemisch dieser

Lösungsmittel mit aliphatischen oder aromatischen Kohlenwasserstoffen wie n-Hexan oder Toluol durchführen. Die Reaktionstemperaturen lassen sich je nach Lösungsmittel zwischen 0 und 120 °C variieren, bevorzugt werden Temperaturen zwischen 30 und 100 °C.

Die hierbei primär entstandenen Magnesiumalkoholate werden sodann durch Hydrolyse mit Wasser oder verdünnten wäßrigen Säuren, wie Salzsäure, Schwefelsäure oder bevorzugt Essigsäure oder besonders bevorzugt mit wäßriger Ammoniumchloridlösung in die Alkohole übergeführt, und diese nach Entfernen der wäßrigen Phase, falls gewünscht, in üblicher Weise durch Extrahieren, Umkristallisieren oder Chromatographieren gereinigt.

Für die stereoselektive Reduktion in der Ausführungsform c) erfolgt die katalytische Hydrierung am besten an einem Edelmetallkontakt. Hierzu sind die Metalle der Platingruppe am besten geeignet. Man kann diese Metalle auf inerten Trägern niederschlagen, wobei ein Metallgehalt von 0,5 bis 10 % im allgemeinen ausreichend ist. Unter Umständen kann man auch die reinen Metalle, zweckmäßig in fein verteilter Form einsetzen. Als besonders geeignet hat sich ein kolloid verteiltes Rutheniumoxidhydrat erwiesen, das durch Fällung aus einer wäßrigen Rutheniumtrichloridlösung beim pH = 8 gewonnen wurde. Die oxidische Form geht unter den Reaktionsbedingungen in den aktiven Katalysator über.

Als Lösungsmittel dienen vorzugsweise Ether wie Dioxan, Tetrahydrofuran oder Glycoldimethylether.

Die Temperatur läßt sich in weiten Grenzen verändern. Als besonders geeignet erwiesen sich Temperaturen zwischen 100 und 150 °C. Bei diesen Temperaturen muß man unter Druck arbeiten ; es genügen Drucke von 10 bis 30 bar. Unter höheren Drucken leidet die Selektivität.

Die Kontaktmenge ist nicht kritisch und hängt neben der Aktivität von der Reaktionstemperatur und dem Wasserstoffpartialdruck ab.

Zur Isolierung der neuen Verbindungen wird nach üblichen Methoden vorgegangen. Im allgemeinen bedürfen die anfallenden Produkte keiner weiteren Reinigung, sie können aber nach bekannten Methoden wie Umkristallisation, Extraktion, Destillation oder Chromatographie weiter gereinigt werden.

Die Ketone der Formel II, die als Ausgangsverbindungen zur Herstellung der Azole der Formel I gemäß Anspruch 1 benötigt werden, lassen sich herstellen, indem man bekannte Ketone der Formel V (DE-OS 26 38 470) oder deren Alkalienolate mit omega-Aryloxyalkenyl- oder -alkinylhalogeniden der Formel VI, gegebenenfalls in Gegenwart einer Base und gegebenenfalls eines Lösungs- oder Verdünnungsmittels alkyliert.

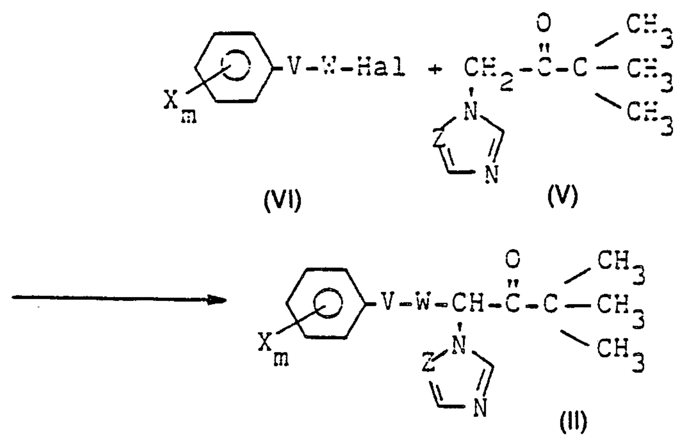

(VI) (V)

(II)

Hierzu können die Ketone V zunächst zu den Alkalienolaten metalliert werden, indem man sie vorzugsweise in Gegenwart eines polaren aprotischen Lösungsmittels wie Dimethylformamid, Acetonitril oder Tetrahydrofuran mit 0,8 bis 1,2 Äquivalenten, bevorzugt 1,0 Äquivalenten, eines Metallierungsreagens wie Natriumhydrid, Lithiumdiisopropylamid oder n-Butyllithium bei 0 bis 100 °C, vorzugsweise bei 10 bis 50 °C umsetzt. Nach anschließender Zugabe von 0,8 bis 2,0, bevorzugt 1,0 Äquivalenten des jeweiligen omega-Aryloxyalkenyl- oder -alkinylhalogenides der Formel VI erhält man bei Reaktionstemperaturen zwischen 0 und 100 °C, bevorzugt bei 5 bis 30 °C die Ketone der Formel II.

Eine Variante dieses Verfahrens besteht darin, daß man die Ketone V in Gegenwart von 0,8 bis 1,2, bevorzugt 1,0 Äquivalenten einer Base, z. B. Kalium-tert.-butoxid, Natriummethoxid oder Kaliumhydroxid mit den omega-Aryloxyalkenyl- oder -alkinylhalogeniden VI umsetzt, wobei man zweckmäßig in Gegenwart eines Lösungs- oder Verdünnungsmittels bei Temperaturen zwischen 0 und 100 °C, bevorzugt bei 5 bis 50 °C, arbeitet.

Als Lösungsmittel oder Verdünnungsmittel kommen hier wiederum polare aprotische Lösungsmittel in Frage, aber auch Alkohole wie Methanol oder tert.-Butanol.

Die omega-Aryloxyalkenyl- oder -alkinylhalogenide VI sind entweder bekannte Verbindungen oder können leicht nach bekannten Verfahren hergestellt werden, z. B. durch Monoalkylierung von Phenolen oder Thiophenolen mit ungesättigten Dihalogenkohlenwasserstoffen z. B. mit 1,4-Dibrombuten-(2) oder 1,4-Dichlorbuten-(2) (vgl. Houben-Weyl, Methoden der Organischen Chemie, Band 6/3, S. 54-59, Thieme-Verlag, Stuttgart 1965).

Falls gewünscht, können die neuen Verbindungen der Formel I auch in Salze mit anorganischen oder organischen Säuren übergeführt werden, wie beispielsweise in Salze der Salzsäure, Bromwasserstoffsäuren, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions beliebig ist.

Ferner lassen sich die Verbindungen der Formel I nach bekannten Methoden in Metallkomplexe überführen. Das kann durch Umsetzung dieser Verbindungen mit geeigneten Metallsalzen wie beispielsweise Kupfer (II)-chlorid, Zink (II)-chlorid, Eisen (III)-chlorid, Kupfer (II)-nitrat, Mangan (II)-chlorid oder Nickel (II)-bromid erfolgen.

Die Herstellung der neuen Verbindungen der Formel I und der Vorprodukte wird durch folgende Beispiele und Vorschriften erläutert :

Vorschrift 1

Herstellung von trans-1,4-Dibrombuten-(2)

618 ml (7,5 mol) cis-1,4-Butendiol, gelöst in 1 000 ml Methylenchlorid, enthaltend 5 ml Pyridin, werden unter Eiskühlung mit 500 ml (5,4 mol) Phosphortribromid tropfenweise versetzt. Nach Abklingen der exothermen Reaktion wird 2 h nachgerührt und mit 1,5 l Eiswasser vermischt. Die abgetrennte organische Phase wird einmal mit Natriumhydrogencarbonat-Lösung und dreimal mit Wasser gewaschen, mit Natriumsulfat getrocknet und im Vakuum vom Lösungsmittel befreit. Zur Isomerisierung der Dibromverbindung zur Transverbindung wird mit 0,5 g Iod bei 150 °C 30 Min. lang gerührt. Anschließende fraktionierte Destillation liefert 1 540 g trans-1,4-Dibrombuten (2).

Vorschrift 2

Herstellung von trans-1-(2-Fluorphenoxy)-4-brombuten-(2)

100 g (0,9 mol) 2-Fluorphenol und 477 g (2,7 mol) trans-1,4-Dibrombuten-(2) werden mit 70 g $K_2CO_3$ in 500 ml Aceton 5 h bei Rückflußtemperatur gerührt. Man filtriert, dampft das Aceton im Vakuum ab, nimmt in Methylenchlorid auf und wäscht viermal mit 1 n wäßriger NaOH-Lösung. Die mit Natriumsulfat getrocknete Methylenchloridphase wird im Vakuum von unumgesetzten 1,4-Dibrombuten befreit. Es verbleiben 175 g trans-1-(2-Fluorphenoxy)-4-brombuten-(2).

Vorschrift 3

Herstellung der Verbindung A

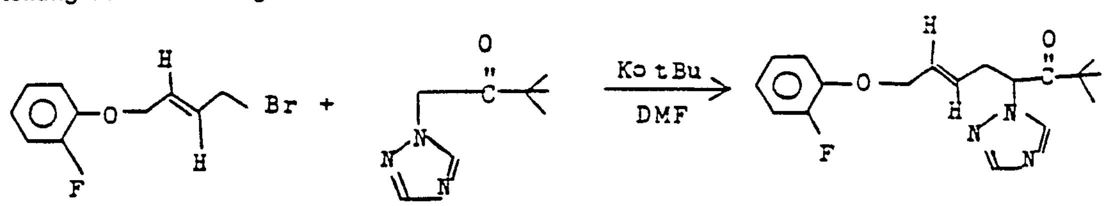


Verbindung A

15,7 g (0,09 mol) Triazolylpinakolon werden in 200 ml Dimethylformamid (DMF) portionsweise mit 11,2 g (0,1 mol) Kaliumtertiärbutylat (KO t Bu) versetzt. Nach etwa 30 Min. tropft man 23 g (0,09 mol) trans-1-(2-Fluorphenoxy)-4-brombuten-(2) zu und rührt 3 h. Nach Filtration wird DMF abgedampft, der Rückstand in Methylenchlorid aufgenommen, mit Wasser gewaschen und mit $NaSO_4$ getrocknet. Das durch Abdampfen des Lösungsmittels erhaltene Rohprodukt wird durch Chromatographie an Kieselgel mit Methylenchlorid als Elutionsmittel gereinigt. Man erhält 25 g Verbindung A als Öl.

Beispiel 1

Herstellung der Verbindung 5

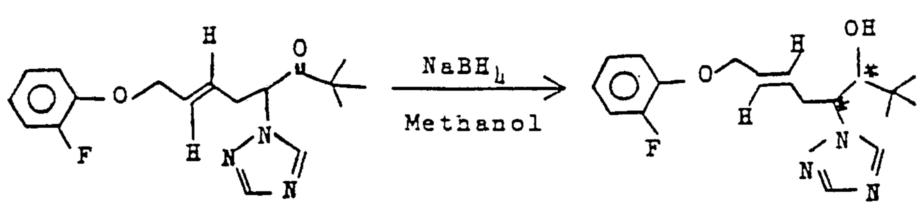


$R^*R^*$ = Dia A

Verbindung A

Verbindung Nr. 5

35 g (0,104 mol) der Verbindung A werden in 200 ml Methanol durch portionsweise Zugabe von 4,1 g (0,104 mol) Natriumborhydrid reduziert. Man rührt 2 h bei ca. 20 °C, dampft in Vakuum ein und nimmt in Methylenchlorid auf. Man schüttelt zweimal mit Wasser aus, trocknet über Natriumsulfat und dampft ein und erhält so 32 g der Verbindung Nr. 5 als Öl, das zu mehr als 90 % diastereomerenrein ist (Dia A = R*,R*-Diastereomeres).

Beispiel 2

Herstellung der Verbindung 6

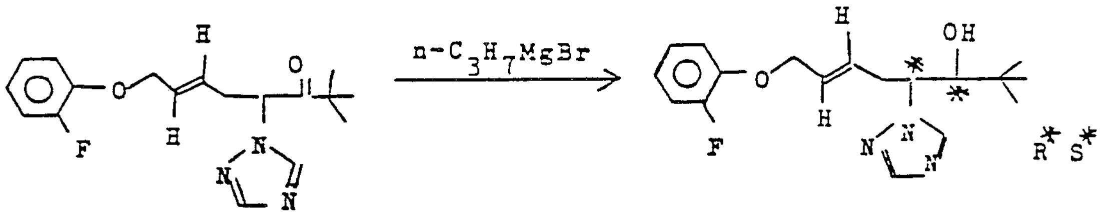


Verbindung A — Verbindung Nr. 6

Aus n-Propylbromid und Mg werden in 50 ml Diethylether in üblicher Weise 0,06 mol n-Propylmagnesiumbromid erzeugt. 10 g (0,03 mol) Verbindung A, gelöst in 100 ml Toluol, werden schnell zugetropft. Nach 1 h setzt man 200 ml wäßrige Ammoniumchloridlösung zu, trennt die Phasen und befreit die mit $Na_2SO_4$ getrocknete organische Phase im Vakuum vom Lösungsmittel. Kristallisation mit Diisopropylether führt nach Absaugen und Trocknen zu 9 g der Verbindung Nr. 6, die zu mehr als 90 % aus dem R*,S*-Diastereomeren (= Dia B) besteht.

In entsprechender Weise können die in der folgenden Tabelle aufgeführten Verbindungen erhalten werden.

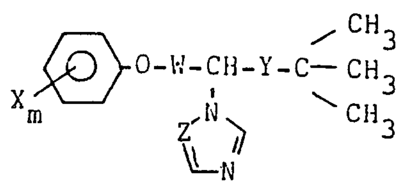

| Verbindung Nr. | $X_m$ | Z | W | Y | Fp [°C] |
|---|---|---|---|---|---|
| 2 | H | N | $CH_2$-CH=CH-$CH_2$ (trans) | CHOH (Dia A) | Öl |
| 3 | H | N | $CH_2$-CH=CH-$CH_2$ (trans) | CHOH (Dia B) | 126 - 128 |
| 5 | 2-F | N | $CH_2$-CH=CH-$CH_2$ (trans) | CHOH (Dia A) | Öl |
| 6 | 2-F | N | $CH_2$-CH=CH-$CH_2$ (trans) | CHOH (Dia B) | 74 - 76 |
| 8 | 3-F | N | $CH_2$-CH=CH-$CH_2$ (trans) | CHOH (Dia A) | |
| 9 | 3-F | N | $CH_2$-CH=CH-$CH_2$ (trans) | CHOH (Dia B) | |
| 11 | 4-F | N | $CH_2$-CH=CH-$CH_2$ (trans) | CHOH (Dia A) | |
| 12 | 4-F | N | $CH_2$-CH=CH-$CH_2$ (trans) | CHOH (Dia B) | |
| 14 | 2-$CH_3$ | N | $CH_2$-CH=CH-$CH_2$ (trans) | CHOH (Dia A) | |
| 15 | 2-$CH_3$ | N | $CH_2$-CH=CH-$CH_2$ (trans) | CHOH (Dia B) | |
| 17 | 3-$CH_3$ | N | $CH_2$-CH=CH-$CH_2$ (trans) | CHOH (Dia A) | |
| 18 | 3-$CH_3$ | N | $CH_2$-CH=CH-$CH_2$ (trans) | CHOH (Dia B) | |
| 20 | 4-$CH_3$ | N | $CH_2$-CH=CH-$CH_2$ (trans) | CHOH (Dia A) | |
| 21 | 4-$CH_3$ | N | $CH_2$-CH=CH-$CH_2$ (trans) | CHOH (Dia B) | |
| 23 | 2-Cl | N | $CH_2$-CH=CH-$CH_2$ (trans) | CHOH (Dia A) | |
| 24 | 2-Cl | N | $CH_2$-CH=CH-$CH_2$ (trans) | CHOH (Dia B) | |
| 26 | 3-Cl | N | $CH_2$-CH=CH-$CH_2$ (trans) | CHOH (Dia A) | |
| 27 | 3-Cl | N | $CH_2$-CH=CH-$CH_2$ (trans) | CHOH (Dia B) | |
| 29 | 4-Cl | N | $CH_2$-CH=CH-$CH_2$ (trans) | CHOH (Dia A) | |
| 30 | 4-Cl | N | $CH_2$-CH=CH-$CH_2$ (trans) | CHOH (Dia B) | |

(Fortsetzung)

| Verbindung Nr. | $X_m$ | Z | W | Y | Fp [°C] |
|---|---|---|---|---|---|
| 32 | 4-O-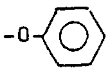 | N | $CH_2$-CH=CH-$CH_2$ (trans) | CHOH (Dia A) | |
| 33 | 4-O-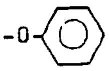 | N | $CH_2$-CH=CH-$CH_2$ (trans) | CHOH (Dia B) | |
| 35 | 4-O-$CH_3$ | N | $CH_2$-CH=CH-$CH_2$ (trans) | CHOH (Dia A) | |
| 36 | 4-O-$CH_3$ | N | $CH_2$-CH=CH-$CH_2$ (trans) | CHOH (Dia B) | |
| 38 | 2-$CF_3$ | N | $CH_2$-CH=CH-$CH_2$ (trans) | CHOH (Dia A) | |
| 39 | 2-$CF_3$ | N | $CH_2$-CH=CH-$CH_2$ (trans) | CHOH (Dia B) | |
| 41 | 3-$CF_3$ | N | $CH_2$-CH=CH-$CH_2$ (trans) | CHOH (Dia A) | |
| 42 | 3-$CF_3$ | N | $CH_2$-CH=CH-$CH_2$ (trans) | CHOH (Dia B) | |
| 44 | 4-$CF_3$ | N | $CH_2$-CH=CH-$CH_2$ (trans) | CHOH (Dia) | |
| 45 | 4-$CF_3$ | N | $CH_2$-CH=CH-$CH_2$ (trans) | CHOH (Dia B) | |
| 47 | 4-Br | N | $CH_2$-CH=CH-$CH_2$ (trans) | CHOH (Dia A) | |
| 48 | 4-Br | N | $CH_2$-CH=CH-$CH_2$ (trans) | CHOH (Dia B) | |
| 49 | 4-tBu | N | $CH_2$-CH=CH-$CH_2$ (trans) | CHOH (Dia B) | |
| 87 | H | N | -$CH_2$-CH=C($CH_3$)$CH_2$- (trans) | CHOH (Dia A) | |
| 88 | H | N | -$CH_2$-CH=C($CH_3$)$CH_2$- (trans) | CHOH (Dia B) | |
| 90 | H | N | -$CH_2$CCl=CCl$CH_2$- (trans) | CHOH (Dia A) | |
| 91 | H | N | -$CH_2$CCl=CCl$CH_2$- (trans) | CHOH (Dia B) | |
| 93 | 2-F | N | -$CH_2$CH=C($CH_3$)$CH_2$- (trans) | CHOH (Dia A) | |
| 94 | 2-F | N | -$CH_2$CH=C($CH_3$)$CH_2$- (trans) | CHOH (Dia B) | |
| 96 | 2-F | N | -$CH_2$CCl=CCl$CH_2$- (trans) | CHOH (Dia A) | |
| 97 | 2-F | N | -$CH_2$CCl=CCl$H_2$- (trans) | CHOH (Dia B) | |
| 99 | 4-F | N | -$CH_2$CCl=CCl$CH_2$- (trans) | CHOH (Dia A) | |
| 100 | 4-F | N | -$CH_2$CCl=CCl$CH_2$- (trans) | CHOH (Dia B) | |
| 102 | 4-F | N | -$CH_2$C($CH_3$)=C($CH_3$)$CH_2$-(trans) | CHOH (Dia A) | |
| 103 | 4-F | N | -$CH_2$C($CH_3$)=C($CH_3$)$CH_2$-(trans) | CHOH (Dia B) | |
| 105 | H | CH | -$CH_2$CH=CH$CH_2$- (trans) | CHOH (Dia A) | |
| 106 | H | CH | -$CH_2$CH=CH$CH_2$- (trans) | CHOH (Dia B) | |
| 108 | 2-F | CH | $CH_2$-CH=CH-$CH_2$ (trans) | CHOH (Dia A) | |
| 109 | 2-F | CH | $CH_2$-CH=CH-$CH_2$ (trans) | CHOH (Dia B) | |
| 111 | 2-Cl | CH | $CH_2$-CH=CH-$CH_2$ (trans) | CHOH (Dia A) | |
| 112 | 2-Cl | CH | $CH_2$-CH=CH-$CH_2$ (trans) | CHOH (Dia B) | |
| 114 | 4-Cl | CH | $CH_2$-CH=CH-$CH_2$ (trans) | CHOH (Dia A) | |
| 115 | 4-Cl | CH | $CH_2$-CH=CH-$CH_2$ (trans) | CHOH (Dia B) | |
| 117 | 2-Cl | N | $CH_2$CH=CH$CH_2$. cis/trans Gem. | CHOH (Dia A) | Öl |
| 118 | 2-Cl | N | $CH_2$CH=CH$CH_2$ cis/trans Gem. | CHOH (Dia B) | |
| 120 | 3-Cl | N | $CH_2$CH=CH$CH_2$ cis/trans Gem. | CHOH (Dia A) | Öl |
| 121 | 3-Cl | N | $CH_2$CH=CH$CH_2$ cis/trans Gem. | CHOH (Dia B) | |
| 123 | 4-Cl | N | $CH_2$CH=CH$CH_2$ cis/trans Gem. | CHOH (Dia A) | Öl |
| 124 | 4-Cl | N | $CH_2$CH=CH$CH_2$ cis/trans Gem. | CHOH (Dia B) | |
| 126 | 2-$CH_3$ | N | $CH_2$CH=CH$CH_2$ cis/trans Gem. | CHOH (Dia A) | Öl |
| 127 | 2-$CH_3$ | N | $CH_2$CH=CH$CH_2$ cis/trans Gem. | CHOH (Dia B) | |
| 129 | 3-$CH_3$ | N | $CH_2$CH=CH$CH_2$ cis/trans Gem. | CHOH (Dia A) | Öl |
| 130 | 3-$CH_3$ | N | $CH_2$CH=CH$CH_2$ cis/trans Gem. | CHOH (Dia B) | |
| 132 | 4-$CH_3$ | N | $CH_2$CH=CH$CH_2$ cis/trans Gem. | CHOH (Dia A) | Öl |
| 133 | 4-$CH_3$ | N | $CH_2$CH=CH$CH_2$ cis/trans Gem. | CHOH (Dia B) | |

(Fortsetzung)

| Verbin-dung Nr. | $X_m$ | Z | W | Y | Fp [°C] |
|---|---|---|---|---|---|
| 135 | 4-O-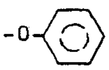 | N | $CH_2CH=CHCH_2$ cis/trans Gem. | CHOH (Dia A) | Öl |
| 136 | 4-O-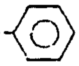 | N | $CH_2CH=CHCH_2$ cis/trans Gem. | CHOH (Dia B) | |
| 138 | H | H | $CH_2-CH=CH-CH_2$ (trans) | CHOH (Dia A) | |
| 139 | H | N | $CH_2-CH=CH-CH_2$ (trans) | CHOH (Dia B) | |
| 141 | 2-F | N | $CH_2-CH=CH-CH_2$ (trans) | CHOH (Dia A) | |
| 142 | 2-F | N | $CH_2-CH=CH-CH_2$ (trans) | CHOH (Dia B) | |
| 144 | $2-CH_3$ | N | $CH_2-CH=CH-CH_2$ (trans) | CHOH (Dia A) | |
| 145 | $2-CH_3$ | N | $CH_2-CH=CH-CH_2$ (trans) | CHOH (Dia B) | |
| 147 | 2-Cl | N | $CH_2-CH=CH-CH_2$ (trans) | CHOH (Dia A) | |
| 148 | 2-Cl | N | $CH_2-CH=CH-CH_2$ (trans) | CHOH (Dia B) | |
| 150 | 3-Cl | N | $CH_2-CH=CH-CH_2$ (trans) | CHOH (Dia A) | |
| 151 | 3-Cl | N | $CH_2-CH=CH-CH_2$ (trans) | CHOH (Dia B) | |
| 153 | $3-CF_3$ | N | $CH_2-CH=CH-CH_2$ (trans) | CHOH (Dia A) | |
| 154 | $3-CF_3$ | N | $CH_2-CH=CH-CH_2$ (trans) | CHOH (Dia B) | |
| 156 | 4-F | N | $CH_2-CH=CH-CH_2$ (trans) | CHOH (Dia A) | |
| 157 | 4-F | N | $CH_2-CH=CH-CH_2$ (trans) | CHOH (Dia B) | |
| 159 | 4-Cl | N | $CH_2-CH=CH-CH_2$ (trans) | CHOH (Dia A) | |
| 160 | 4-Cl | N | $CH_2-CH=CH-CH_2$ (trans) | CHOH (Dia B) | |
| 162 | H | CH | $CH_2-CH=CH-CH_2$ (trans) | CHOH (Dia A) | |
| 163 | H | CH | $CH_2-CH=CH-CH_2$ (trans) | CHOH (Dia B) | |
| 165 | 4-F | CH | $CH_2-CH=CH-CH_2$ (trans) | CHOH (Dia A) | |
| 166 | 4-F | CH | $CH_2-CH=CH-CH_2$ (trans) | CHOH (Dia B) | |

Fußnote :

Dia A : bedeutet bevorzugt (> 85 %) oder ausschließlich Diastereomer A mit R*,R*-Konfiguration

Dia B : bedeutet bevorzugt (> 85 %) oder ausschließlich Diastereomer B mit R*,S*-Konfiguration

Mit einem cis/trans-Gemisch ist ein Gemisch von Doppelbindungsisomeren gemeint, das 40-60 % des einen und entsprechend 60-40 % des anderen Isomeren enthält.

Die neuen Verbindungen werden als fungizide Wirkstoffe angewendet, indem man die Substrate bzw. Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Wirkstoffe dieser Erfindung können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Ketone (z. B. Cyclohexanon, Amine (z. B. Ethanolamin, Dimethylformamid) und Wasser ; Trägerstoffe wie natürliche Gesteinsmehle, z. B. Kaoline, Tonerden, Talkum, Kreide und synthetische Gesteinsmehle (z. B. hochdisperse Kieselsäure, Silikate) ; Emulgiermittel, wie nichtionogene und anionische Emulgatoren (z. B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die Wirkstoffe können auch im Materialschutz u. a. zur Bekämpfung holzzerstörender Pilze wie Coniophora puteana und Polystictus versicolor eingesetzt werden. Die Wirkstoffe können auch

als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind :

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 3 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 6 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

VI. 3 Gewichtsteile der Verbindung Nr. 2 werden mit 97 Gewichtsteilen feinteiligem Kaolin innig vermischt. Man erhält auf diese Weise ein Stäubemittel, das 3 Gew.% des Wirkstoffs enthält.

VII. 40 Gewichtsteile der Verbindung Nr. 3 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit Wasser erhält man eine wäßrige Dispersion.

VIII. 20 Teile der Verbindung Nr. 6 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, z. B. Herbiziden, Insektiziden, Wachstumsrergulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die Wirkstoffe dieser Erfindung kombiniert werden können, soll die Kombinationsmöglichkeiten erläutern, nicht aber einschränken :

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,
heterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol

2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethyl-phthalimid
N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Jod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie

Dodecylguanidinacetat
3-(3-(3,5-Dimethyl-2-oxycyclohexyl)-2-hydroxyethyl)-gluatarimid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl)-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl) benzhydrylalkohol

Die Wirkstoffe dieser Erfindung und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten, Basidiomyceten und Deuteromyceten, aus. Sie sind zum Teil systemisch wirksam und können als Blatt- und Bodenfungizide eingesetzt werden.

Besonders interessant sind die Fungizide dieser Erfindung für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Garten- und Weinbau, sowie Gemüse - wie Gurken, Bohnen und Kürbisgewächse.

Die Wirkstoffe dieser Erfindung sind insbesondere geeignet zur Bekämpfung folgender Pflanzenkrankheiten :

Erysiphe graminis (echter Mehltau) in Getreide,

Erysiphe cichoracearum an Kürbisgewächsen,
Podosphaera leucotricha an Äpfeln,
Uncinula necator an Reben,
Puccinia-Arten an Getreide,
Rhizoctonia solani an Baumwolle,
Ustilago-Arten an Getreide und Zuckerrhor,
Venturia inaequalis (Schorf) an Äpfeln,
Septoria nodorum an Weizen,
Botrytis cinerea (Grauschimmel) an Erdbeeren, Reben,
Cercospora musae an Bananen,
Pseudocercosporella herpotrichoides an Weizen, Gerste,
Pyricularia oryzae an Reis,
Hemileia vastatrix an Kaffee,
Alternaria solani an Kartoffeln, Tomaten,
Verticillium an Baumwolle und Gemüse,
Peronospora an Reben.

Besonders hervorzuheben ist die Wirksamkeit gegen Botrytis und Alternaria.

Die folgenden Versuche erläutern die fungizide Wirkung. Der bekannte Wirkstoff 1-[2-(2,4-Dichlorphenyl)-2-(2-propenyloxy)-ethyl]-1H-imidazol (A) wurde zum Vergleich verwendet.

Versuch 1

Wirksamkeit gegen Weizenmehltau

Blätter von in Töpfen gewachsenen Weizensämlingen der Sorte « Jubilar » werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22 °C und 75 bis 80 % relativer Luftfeuchtigkeit aufgestellt. Nach 7 Tagen wird das Ausmaß der Mehltauentwicklung ermittelt.

Das Ergebnis des Versuches zeigt, daß beispielsweise bei der Anwendung als 0,025 %ige Wirkstoffbrühe die Verbindungen 3 und 6 eine bessere fungizide Wirkung zeigen (beispielsweise 100 %) als der bekannte Wirkstoff A (97 %).

Versuch 2

Wirksamkeit gegen Weizenbraunrost

Blätter von in Töpfen gewachsenen Weizensämlingen der Sorte « Jubilar » werden mit Sporen des Braunrostes (Puccinia recondita) bestäubt. Danach werden die Töpfe für 24 Stunden bei 20 bis 22 °C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22 °C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß beispielsweise bei der Anwendung als 0,025 %ige Wirkstoffbrühe die Verbindungen 2, 3 und 6 eine bessere fungizide Wirkung zeigten (beispielsweise 100 %) als der bekannte Wirkstoff A (beispielsweise 70 %).

Versuch 3

Wirksamkeit gegen Botrytis cinerea an Paprika

Paprikasämlinge der Sorte « Neusiedler Ideal Elite » werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24 °C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise bei der Anwendung als 0,05 %ige Wirkstoffbrühe die Verbindungen 2, 3 und 6 eine bessere fungizide Wirkung zeigten (beispielsweise 97 %) als der bekannte Wirkstoff A (beispielsweise 70 %).

**Patentansprüche**

1. Azolverbindung der Formel

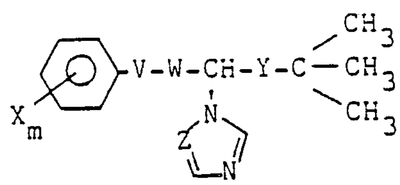

(I)

in welcher
V für Sauerstoff steht,
X für Wasserstoff, Fluor, Chlor, Brom $C_1$-$C_4$-Alkyl, $C_1$-$C_4$-Alkoxy, Trifluormethyl, Phenyl oder Phenoxy steht,
m eine ganze Zahl von 1 bis 5 ist, wobei die einzelnen Atome oder Gruppen gleich oder verschieden sind, wenn m größer als 1 ist,
W für eine Gruppe der Formel $CH_2$—$CR^1$=$CR^2$—$CH_2$ steht, wobei $R^1$ und $R^2$ gleich oder verschieden sind und Wasserstoff, Methyl, Ethyl, Chlor oder Fluor bedeuten,
Z für CH oder N steht,
Y für die Gruppe CHOH steht,
sowie deren für Pflanzen verträgliche Säureadditionssalze und Metall-Komplexe.

2. Fungizides Mittel, enthaltend einen inerten Zusatzstoff und eine Azolverbindung der Formel

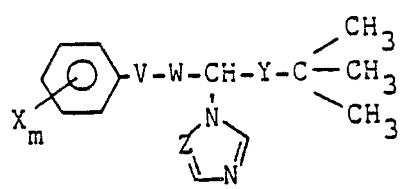

(I)

in welcher
V für Sauerstoff steht,
X für Wasserstoff, Fluor, Chlor, Brom $C_1$-$C_4$-Alkyl, $C_1$-$C_4$-Alkoxy, Trifluormethyl, Phenyl oder Phenoxy steht,
m eine ganze Zahl von 1 bis 5 ist, wobei die einzelnen Atome oder Gruppen gleich oder verschieden sind, wenn m größer als 1 ist,
W für eine Gruppe der Formel $CH_2$—$CR^1$=$CR^2$—$CH_2$ steht, wobei $R^1$ und $R^2$ gleich oder verschieden sind und Wasserstoff, Methyl, Ethyl, Chlor oder Fluor bedeuten,
Z für CH oder N steht,
Y für die Gruppe CHOH steht,
sowie deren für Pflanzen verträgliche Säureadditionssalze und Metall-Komplexe.

3. Verfahren zur Herstellung eines fungiziden Mittels, dadurch gekennzeichnet, daß man einen inerten Zusatzstoff vermischt mit Azolverbindungen der Formel I gemäß Anspruch 1.

4. Verfahren zur Bekämpfung von Pilzen, dadurch gekennzeichnet, daß man die Pilze oder die von Pilzbefall bedrohten Materialien, Flächen, Pflanzen oder Saatgüter behandelt mit einer fungizid wirksamen Menge einer Azolverbindung der Formel

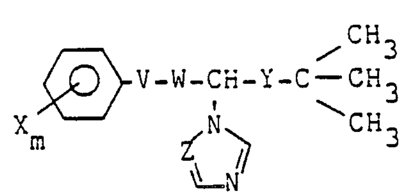

(I)

in welcher
V für Sauerstoff steht,
X für Wasserstoff, Fluor, Chlor, Brom $C_1$-$C_4$-Alkyl, $C_1$-$C_4$-Alkoxy, Trifluormethyl, Phenyl oder Phenoxy steht,
m eine ganze Zahl von 1 bis 5 ist, wobei die einzelnen Atome oder Gruppen gleich oder verschieden sind, wenn m größer als 1 ist,
W für eine Gruppe der Formel $CH_2$—$CR^1$=$CR^2$—$CH_2$ steht, wobei $R^1$ und $R^2$ gleich oder verschieden sind und Wasserstoff, Methyl, Ethyl, Chlor oder Fluor bedeuten,
Z für CH oder N steht,

Y für die Gruppe CHOH steht,
oder deren für Pflanzen verträglichem Säureadditionssalz oder Metallkomplex.

5. Verbindung der Formel I gemäß Anspruch 1, dadurch gekennzeichnet, daß
V Sauerstoff
X Wasserstoff oder Fluor
W die Gruppe $—CH_2—CH=CH—CH_2—$
Z Stickstoff und
Y CHOH bedeutet.

6. Fungizides Mittel gemäß Anspruch 2, dadurch gekennzeichnet, daß
V Sauerstoff
X Wasserstoff oder Fluor
W die Gruppe $—CH_2—CH=CH—CH_2—$
Z Stickstoff und
Y CHOH bedeutet.

**Claims**

1. An azole compound of the formula

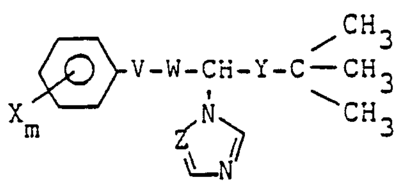

(I)

where
V is oxygen,
X is hydrogen, fluorine, chlorine, bromine, $C_1$-$C_4$-alkyl, $C_1$-$C_4$-alkoxy, trifluoromethyl, phenyl or phenoxy,
m is an integer from 1 to 5, the individual atoms or groups being identical or different when m is greater than 1,
W is a group of the formula $CH_2—CR^1=CR^2—CH_2$, where $R^1$ and $R^2$ are identical or different and are each hydrogen, methyl, ethyl, chlorine or fluorine,
Z is CH or N, and
Y is CHOH,
or a plant-tolerated acid addition salt or metal complex thereof.

2. A fungicide containing an inert additive and an azole compound of the formula

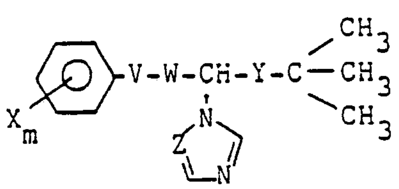

(I)

where
V is oxygen,
X is hydrogen, fluorine, chlorine, bromine, $C_1$-$C_4$-alkyl, $C_1$-$C_4$-alkoxy, trifluoromethyl, phenyl or phenoxy,
m is an integer from 1 to 5, the individual atoms or groups being identical or different when m is greater than 1,
W is a group of the formula $CH_2—CR^1=CR^2—CH_2$, where $R^1$ and $R^2$ are identical or different and are each hydrogen, methyl, ethyl, chlorine or fluorine,
Z is CH or N, and
Y is CHOH,
or a plant-tolerated acid addition salt or metal complex thereof.

3. A process for preparing a fungicide wherein an inert additve is mixed with an azole compound of the formula I as claimed in claim 1.

4. A process for combating fungi, wherein the fungi or the materials, areas, plants or seed threatened by fungus attack are treated with a fungicidally effective amount of an azole compound of the formula

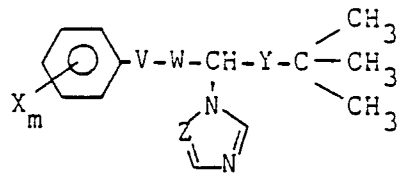

(I)

where

V is oxygen,

X is hydrogen, fluorine, chlorine, bromine, $C_1$-$C_4$-alkyl, $C_1$-$C_4$-alkoxy, trifluoromethyl, phenyl or phenoxy,

m is an integer from 1 to 5, the individual atoms or groups being identical or different when m is greater than 1,

W is a group of the formula $CH_2$—$CR^1$=$CR^2$—$CH_2$, where $R^1$ and $R^2$ are identical or different and are each hydrogen, methyl, ethyl, chlorine or fluorine,

Z is CH or N, and

Y is CHOH,

or a plant-tolerated acid additon salt or metal complex thereof.

5. A compound of the formula I as claimed in claim 1, wherein

V is oxygen,

X is hydrogen or fluorine,

W is —$CH_2$—CH=CH—$CH_2$—,

Z is nitrogen, and

Y is CHOH.

6. Fungicide as claimed in claim 2, wherein

V is oxygen,

X is hydrogen or fluorine,

W is —$CH_2$—CH=CH—$CH_2$—,

Z is nitrogen and

Y is CHOH.

**Revendications**

1. Composé azole de formule

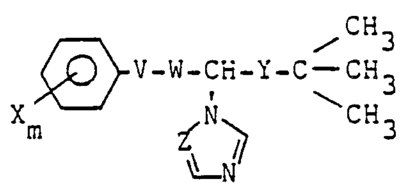

(I)

dans laquelle

V représente oxygène,

X hydrogène, fluor, chlore, brome, alkyle en $C_1$-$C_4$, alcoxy en $C_1$-$C_4$, trifluorométhyle, phényle ou phénoxy,

m est un nombre entier de 1 à 5, les différents atomes ou groupes étant identiques ou différents, lorsque m est supérieur à 1,

W représente un groupe de formule $CH_2$—$CR^1$=$CR^2$—$CH_2$, $R^1$ et $R^2$ étant identiques ou différents et représentant hydrogène, méthyle, éthyle, chlore ou fluor,

Z est mis pour CH ou N

Y représente le groupe CHOH

ainsi que leurs sels d'addition d'acide compatibles pour les plantes et les complexes métalliques.

2. Agent fongicide contenant un additif inerte et un composé azole de formule

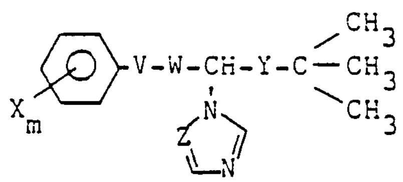

(I)

dans laquelle

V représente oxygène,

X hydrogène, fluor, chlore, brome, alkyle en $C_1$-$C_4$, alcoxy en $C_1$-$C_4$, trifluorométhyle, phényle ou phénoxy,

m est un nombre entier de 1 à 5, les différents atomes ou groupes étant identiques ou différents, lorsque m est supérieur à 1,

W représente un groupe de formule $CH_2—CR^1=CR^2—CH_2$, $R^1$ et $R^2$ étant identique ou différents et représentant hydrogène, méthyle, éthyle, chlore ou fluor,

Z est mis pour CH ou N

Y représente le groupe CHOH

ainsi que leurs sels d'addition d'acide compatibles pour les plantes, et les complexes métalliques.

3. Procédé de préparation d'un agent fongicide, caractérisé par le fait que l'on mélange un additif inerte avec un composé azole de formule I selon la revendication 1.

4. Procédé de lutte contre les champignons, caractérisé par le fait que l'on traite les champignons ou les matériaux, surfaces, plantes ou semences menacés par une attaque par les champignons, avec une quantité efficace du point de vue fongicide d'un composé azole de formule

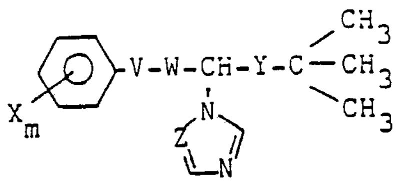

(I)

dans laquelle

V représente oxygène,

X hydrogène, fluor, chlore, brome, alkyle en $C_1$-$C_4$, alcoxy en $C_1$-$C_4$, trifluorométhyle, phényle ou phénoxy,

m est un nombre entier de 1 à 5, les différents atomes ou groupes étant identiques ou différents, lorsque m est supérieur à 1,

ainsi que leurs sels d'addition d'acide compatibles pour les plantes, et les complexes métalliques.

5. Composé de formule 1, selon la revendication 1, caractérisé par le fait que :

V représente oxygène

X hydrogène ou fluor,

W le groupe $—CH_2—CH=CH—CH_2—$

Z azote et

Y CHOH.

6. Agent fongicide selon la revendication 2, caractérisé par le fait que :

V représente oxygène

X hydrogène ou fluor,

W le groupe $—CH_2—CH=CH—CH_2—$

Z azote et

Y CHOH.